# EUROPEAN PATENT APPLICATION

(11) **EP 1 902 692 A1**
(43) Date of publication of application: **26.03.2008**
(21) Application number: 07118503.7
(22) Date of filing: 08.05.2006
(51) Int. Cl.: A61F 5/41, A61F 6/04, A61H 19/00

(54) **Male stimulating contraceptives**

(30) Priority: 06.05.2005 US 678824 P; 06.05.2005 US 678825 P; 21.07.2005 US 700853 P
(62) Divisional of application: 06759353.3
(71) Applicant: Reddy, Alla V. K., Plainsboro, New Jersey 08536 (US); Alla, Ravikumar Reddy, Plainsboro, New Jersey 08536 (US); Alla, Madhusudhan Reddy, Plainsboro, New Jersey 08536 (US); Alla, Raghunatha Reddy, Plainsboro, New Jersey 08536 (US)
(72) Inventor: Reddy, Alla V. K., Plainsboro, New Jersey 08536 (US); Alla, Ravikumar Reddy, Plainsboro, New Jersey 08536 (US); Alla, Madhusudhan Reddy, Plainsboro, New Jersey 08536 (US); Alla, Raghunatha Reddy, Plainsboro, New Jersey 08536 (US)
(74) Representative: Clark, Jane Anne

(57) **Abstract**

A male condom (1010,1310) comprising a first pouch defining a first chamber (1013,1313) and a second pouch defining a second chamber (1073,1373) for receiving a stimulating device.
The second pouch can extend either inwardly into said first chamber or parallel to said first chamber.

## Description

This application claims the benefit of U.S. Provisional Patent Application No. 60/678,824, filed May 6, 2005, of U.S. Provisional Patent Application No. 60/678,825, filed May 6, 2005, and of U.S. Provisional Patent Application No. 60/700,853, filed July 21, 2005, each of which is hereby incorporated by reference herein in its entirety.

### Field of the Invention

This invention relates generally to prophylactic devices and, more particularly, to male condoms.

### Background of the Invention

With the proliferation of sexually transmitted diseases (STDs), including acquired immune-deficiency syndrome (AIDS), there is an increasing need for effective means to prevent the exchange of bodily fluids during sexual intercourse and the resultant transmission of STDs. One method for accomplishing this goal is by introducing an effective barrier between the male and female sex organs during sexual intercourse. A wide variety of male condoms or prophylactic devices are known for creating such barriers. However, the male condoms currently available do not enhance, and rather usually diminish, the pleasure attained by each partner during sexual intercourse.

Accordingly, it would be advantageous to be able to provide a male condom that provides stimulation to a sexual organ of at least one of the partners during sexual intercourse.

### Summary of the Invention

It is an object of this invention to provide a male condom that provides stimulation to a sexual organ of at least one of the partners during sexual intercourse.

In accordance with the invention, there is provided a male condom that includes an open end, a closed end, and a tubular body extending longitudinally between the open end and the closed end to define a chamber. The tubular body has a wall with an interior surface and an exterior surface. The tubular body has at least one curved region to define a long side portion and a short side portion of the wall. The tubular body is capable of forming one or more folds along the long side portion of the wall for stimulating a sexual organ during sexual intercourse on insertion of a penis into the chamber.

### Brief Description of the Drawings

The above and other advantages of the invention will be more apparent upon consideration of the following detailed description, taken in conjunction with the accompanying drawings, in which like reference characters refer to like parts throughout, and in which:

FIG. 1 is a partial cross-sectional view of a male condom according to an embodiment of the invention;

FIG. 2 is a side elevational view of the condom of FIG. 1, taken from line II-II of FIG. 1;

FIG. 3 is a partial cross-sectional view of the condom of FIGS. 1 and 2, with a penis inserted therein, according to invention;

FIG. 4 is a top elevational view of the condom of FIGS. 1-3, taken from line IV-IV of FIG. 3;

FIG. 5 is a fragmentary sectional view of the condom of FIGS. 1-4 showing a pocket stroked inwardly;

FIG. 6 is a partial cross-sectional view of the condom of FIGS. 1-5, similar to FIG. 3, but in a first stage of use within a female's pelvic region, according to the invention;

FIG. 7 is a partial cross-sectional view of the condom of FIGS. 1-6, similar to FIGS. 3 and 6, but in a second stage of use within the pelvic region of FIG. 6, according to the invention;

FIG. 8 is a partial cross-sectional view of the condom of FIGS. 1-7, similar to FIGS. 3, 6, and 7, but in a third stage of use within the pelvic region of FIGS. 6 and 7, according to the invention;

FIG. 9 is a partial cross-sectional view of the condom of FIGS. 1-8, similar to FIGS. 3 and 6-8, but in a fourth stage of use within the pelvic region of FIGS. 6-8, according to the invention;

FIG. 10 is a partial cross-sectional view of a male condom according to a second embodiment of the invention;

FIG. 11 is a side elevational view of the condom of FIG. 10, taken from line XI-XI of FIG. 10;

FIG. 12 is a partial cross-sectional view of the condom of FIGS. 10 and 11, with a penis inserted therein, according to invention;

FIG. 13 is a top elevational view of the condom of FIGS. 10-12, taken from line XIII-XIII of FIG. 12;

FIG. 14 is a partial cross-sectional view of a male condom according to a third embodiment of the invention;

FIG. 15 is a side elevational view of the condom of FIG. 14, taken from line XV-XV of FIG. 14;

FIG. 16 is a partial cross-sectional view of the condom of FIGS. 14 and 15, with a penis inserted therein, according to invention;

FIG. 17 is a top elevational view of the condom of FIGS. 14-16, taken from line XVII-XVII of FIG. 16;

FIG. 18 is a partial cross-sectional view of a male condom according to a fourth embodiment of the invention;

FIG. 19 is a side elevational view of the condom of FIG. 18, taken from line XIX-XIX of FIG. 18;

FIG. 20 is a partial cross-sectional view of the condom of FIGS. 18 and 19, with a penis inserted therein, according to invention;

FIG. 21 is a top elevational view of the condom of FIGS. 18-20, taken from line XXI-XXI of

### FIG. 20;

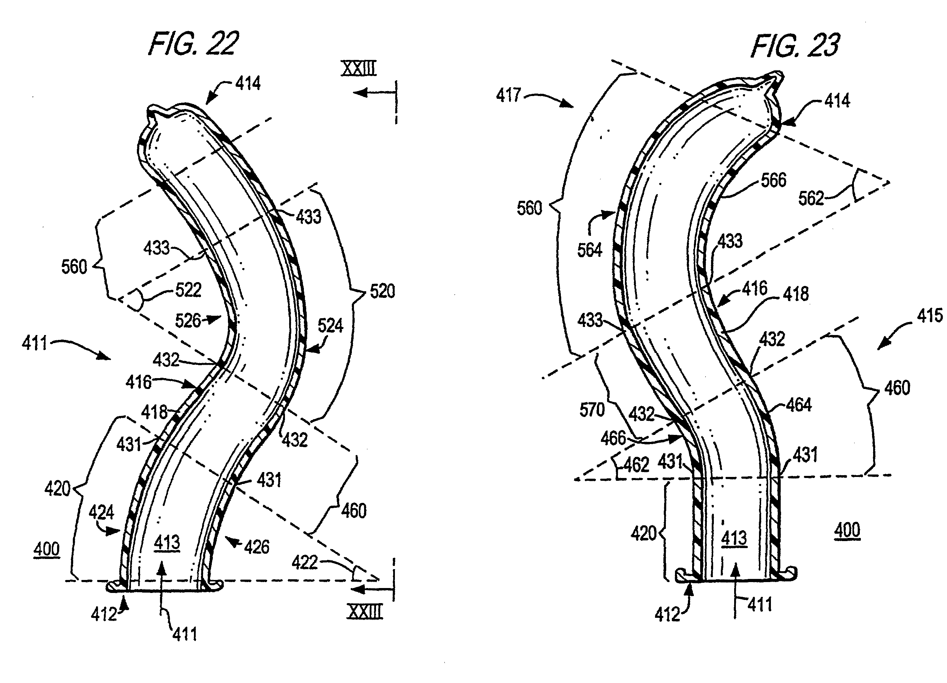

FIG. 22 is a partial cross-sectional view of a male condom according to a fifth embodiment of the invention;

FIG. 23 is a partial cross-sectional view of the male condom of FIG. 22, taken from line XXIII-XXIII of FIG. 22;

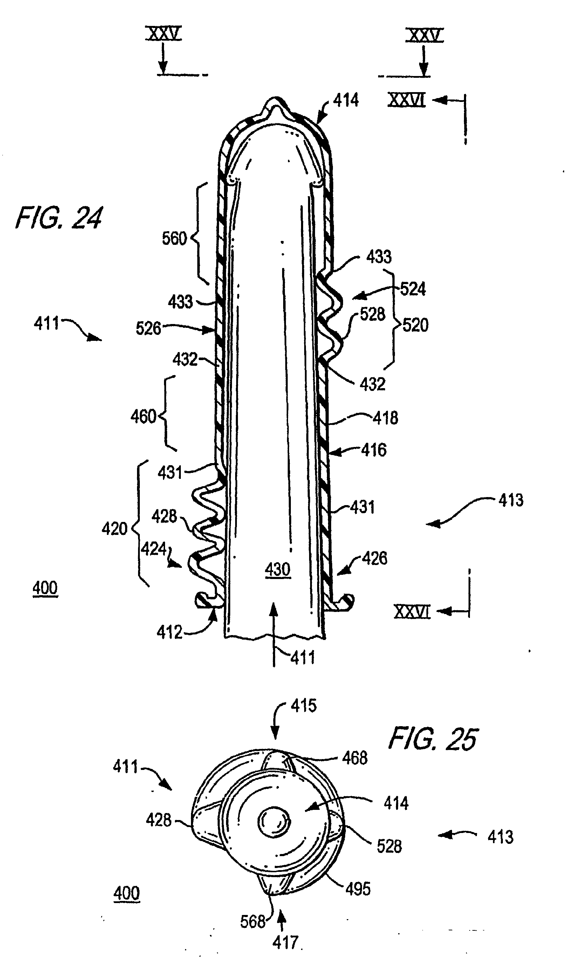

FIG. 24 is a partial cross-sectional view, similar to FIG. 22, of the condom of FIGS. 22 and 23, with a penis inserted therein, according to invention;

FIG. 25 is a top elevational view of the condom of FIGS. 22-24, taken from line XXV-XXV of FIG. 24;

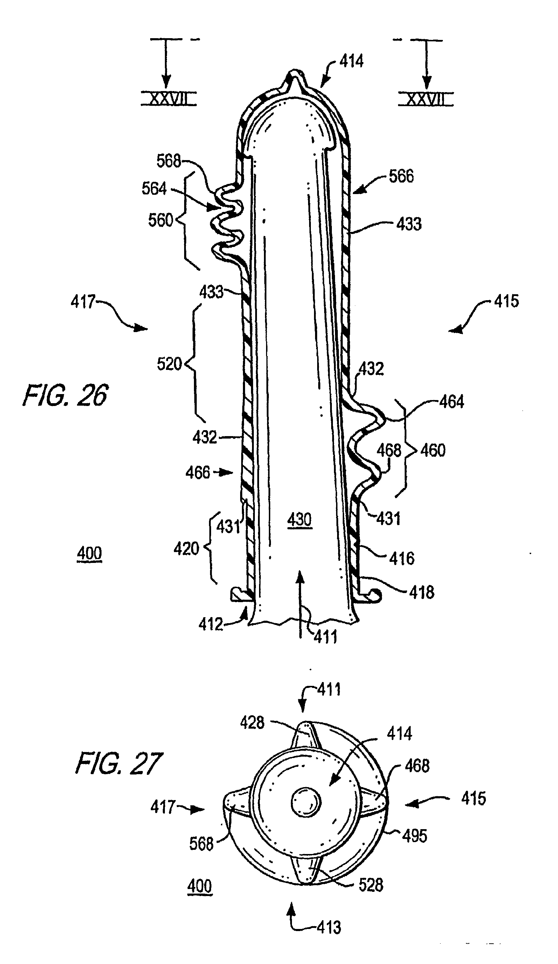

FIG. 26 is a partial cross-sectional view, similar to FIG. 23, of the condom of FIGS. 22-25, with a penis inserted therein, according to invention;

FIG. 27 is a top elevational view of the condom of FIGS. 22-26, taken from line XXVII-XXVII of FIG. 26;

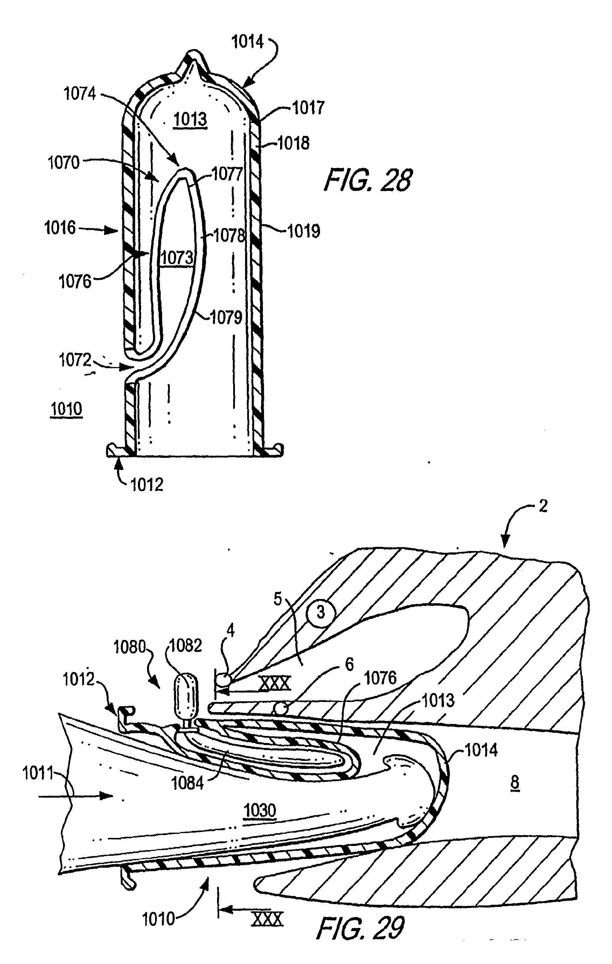

FIG. 28 is a partial cross-sectional view of a male condom according to a sixth embodiment of the invention;

FIG. 29 is a partial cross-sectional view of the condom of FIG. 28, similar to FIG. 28, but in a stage of use within a female's pelvic region, according to the invention;

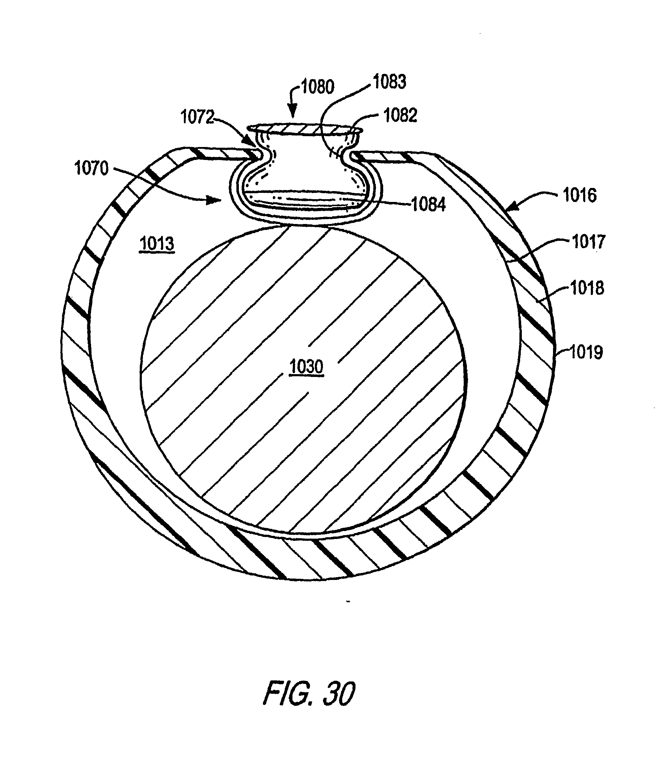

FIG. 30 is a partial cross-sectional view of the condom of FIGS. 28 and 29, taken from line XXX-XXX of FIG. 29;

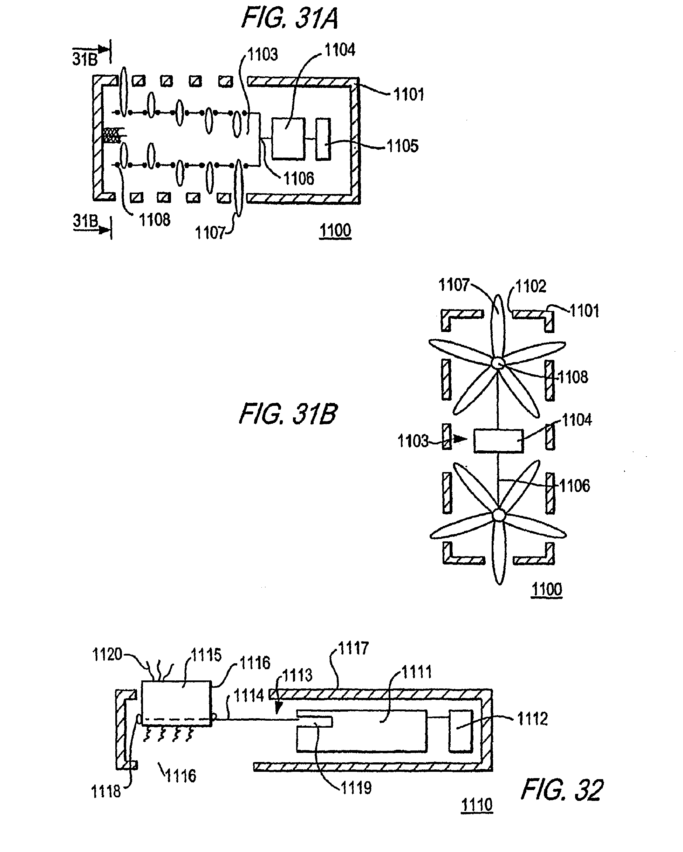

FIG. 31A is a partial cross-sectional view of a first embodiment of a stimulator according to the invention;

FIG. 31B is a partial cross-sectional view of the stimulator of FIG. 31A, taken from line XXXIB-XXXIB of FIG. 31A;

FIG. 32 is a partial cross-sectional view, similar to FIG. 31A, of a second embodiment of a stimulator according to the invention;

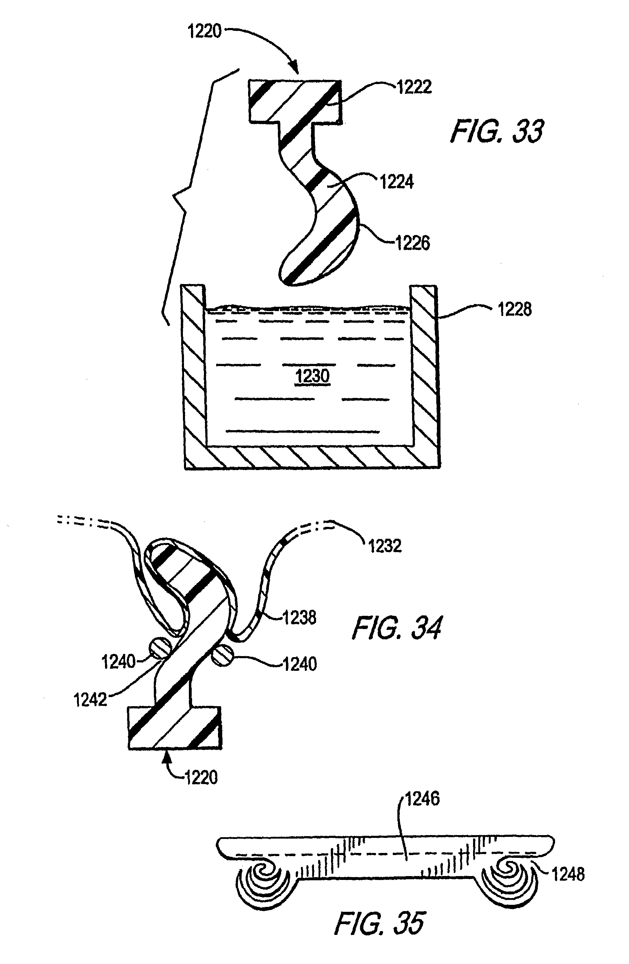

FIG. 33 is a diagrammatic view showing a dipping step in a process according to the invention;

FIG. 34 is a diagrammatic view showing a stripping step in the process of FIG. 33;

FIG. 35 is a diagrammatic view showing a final step in the process of FIGS. 33 and 34;

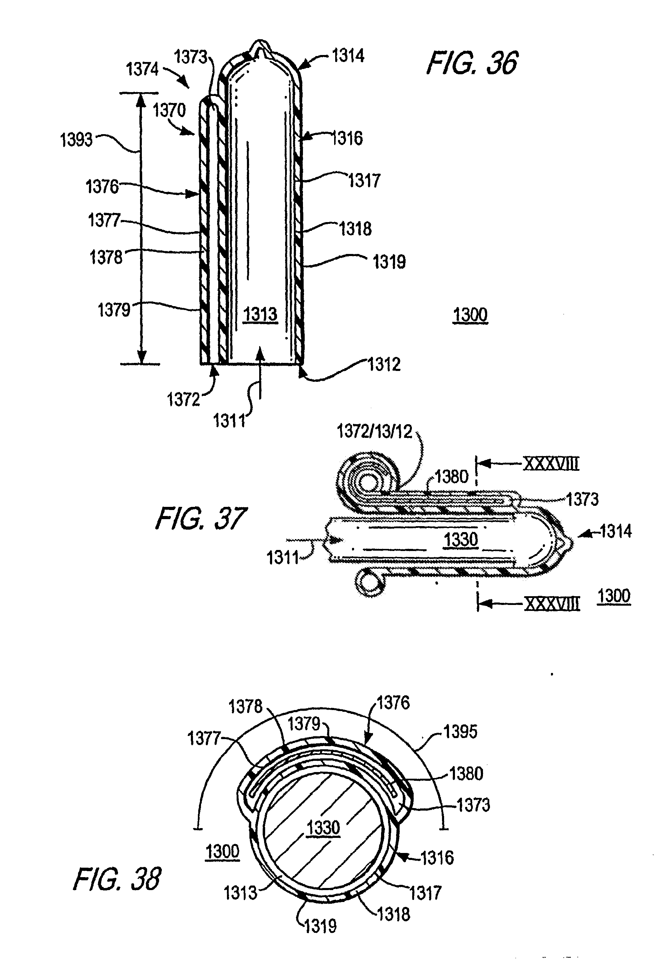

FIG. 36 is a partial cross-sectional view of a male condom according to a seventh embodiment of the invention;

FIG. 37 is a partial cross-sectional view of the condom of FIG. 36, similar to FIG. 36, , with a penis inserted therein, according to invention; and

FIG. 38 is a partial cross-sectional view of the condom of FIGS. 36 and 37, taken from line XXXVIII-XXXVIII of FIG. 37.

### Detailed Description of the Invention

The invention provides a male condom having an open end, a closed end, and a tubular body extending longitudinally between the open end and the closed end to define a chamber. The tubular body has a wall with an interior surface and an exterior surface. The tubular body also has at least one curved region to define a long side portion and a short side portion of the wall. The tubular portion is capable of forming one or more folds along the long side portion of the wall for stimulating a sexual organ during sexual intercourse on insertion of a penis into the chamber.

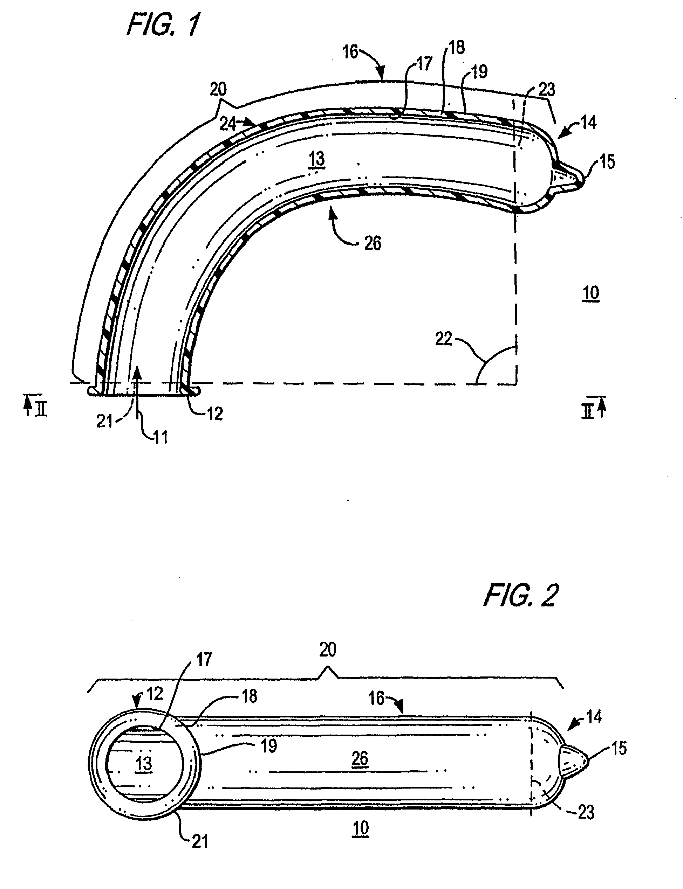

The invention will now be described with reference to FIGS. 1-38.

FIG. 1 illustrates a male condom 10 having an open end 12, a closed end 14, and a tubular body portion 16 that extends longitudinally between open end 12 and closed end 14 to define a chamber 13. Tubular body 16 has a thin membrane wall 18 with an interior surface 17 and an exterior surface 19, and is preferably formed from any suitable elastic and impermeable membrane material, such as natural rubber (e.g., latex), synthetic rubber (e.g., silicone rubber or neoprene), polyurethane, and other elastic substances (e.g., thermoplastic polyolefin elastomers), for example.

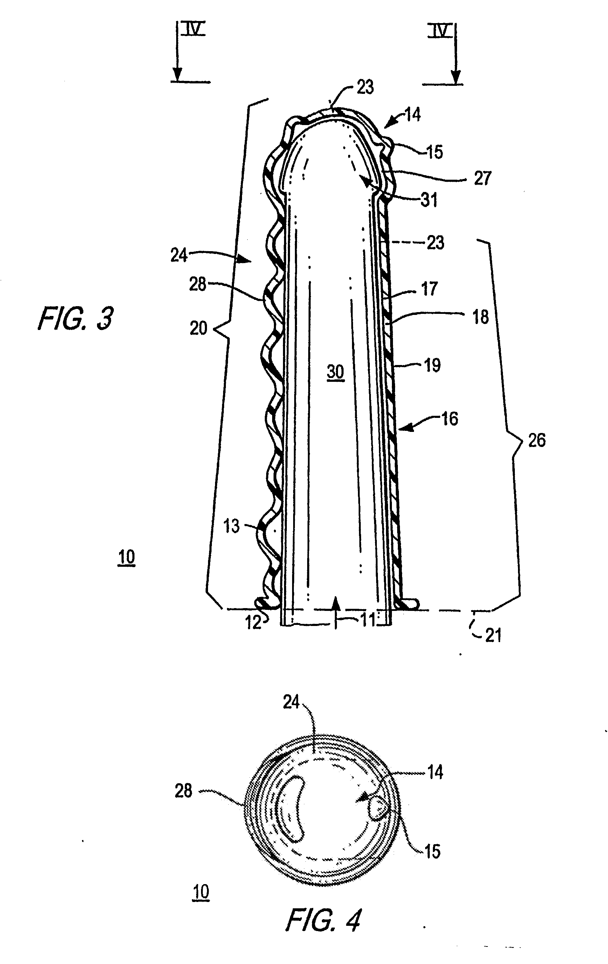

According to certain embodiments, the condom is preferably provided with a curved region along at least a portion of the tubular body as it extends longitudinally between its open end and its closed end. For example, as shown in FIGS. 1 and 2, condom 10 is preferably provided with a curved region 20 along the entire length of tubular body 16 between open end 12 and closed end 14. Curved region 20 has a proximal end 21 (i.e., at open end 12, according to this particular embodiment), a distal end 23 (i.e., at closed end 14, according to this particular embodiment), and a curvature angle 22, thereby defining a long side portion 24 and a short side portion 26 of wall 18, such that when a penis 30 is inserted into chamber 13 through open end 12 in the direction of arrow 11, one or more folds 28 are formed along long side portion 24 of wall 18, as shown in FIGS. 3 and 4, for example.

With continued reference to FIGS. 1 and 2, closed end 14 is preferably dome-shaped to match the shape of the tip of a penis (see, e.g., glans penis 31 of penis 30 in FIG. 3). Closed end 14 is also preferably provided with a reservoir tip or bulge 15 for collecting semen after ejaculation. As shown in FIGS. 3 and 4, the relationship between the length of penis 30, long side portion 24, and short side portion 26 is such that upon insertion of penis 30 into chamber 13, short side portion 26 of wall 18 is preferably pulled taught, while folds 28 are formed along long side portion 24 of wall 18 opposite that of small side portion 26 due to the inequality in lengths of the side portions of the wall.

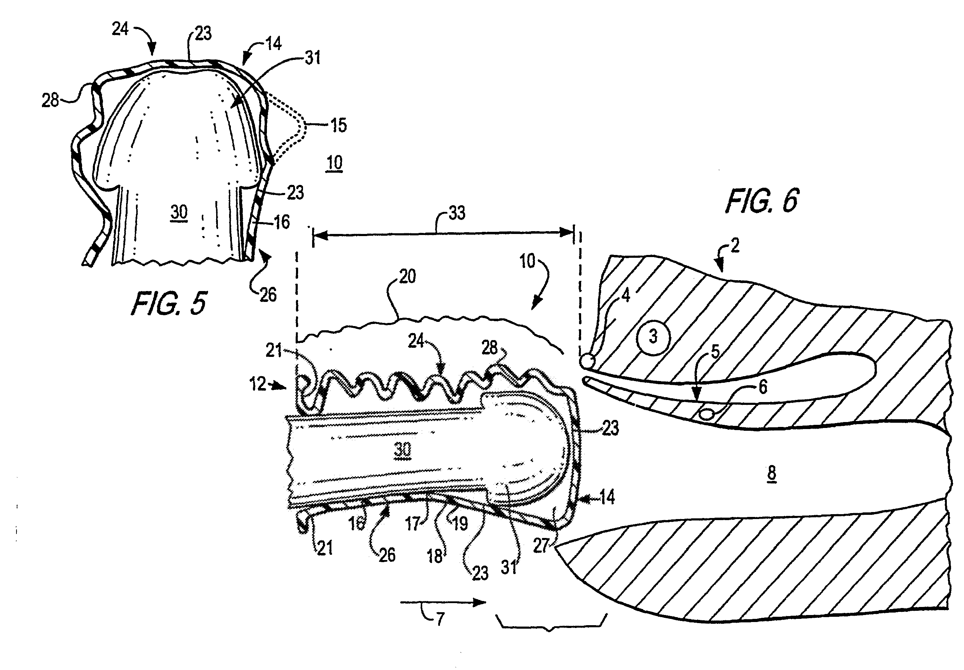

In certain embodiments, the taughtness of short side portion 26 preferably draws at least a portion of closed end 14 towards the side of glans penis 31 adjacent to short side portion 26, as shown in FIGS. 3 and 4, such that at least a portion of closed end 14 and/or bulge 15 forms a pocket 27 in one side of condom 10 at a point overlying and in spaced relationship to a portion of glans penis 31. As a consequence of pocket 27, condom 10 does not bind glans penis 31 so as to dull the sensitivity of the glans penis, and thereby reduce the acceptability of condom use. Instead, due to the taughtness of short side portion 26, the portion of closed end 14 and/or bulge 15 that forms pocket 27 may move forward and backward during sexual intercourse, as shown in the enlarged fragmentary sectional view thereof in FIG. 5. The back and forth movement will stimulate glans penis 31 so as to provide enhanced sensation to the male user of condom 10 during sexual intercourse. Pocket 27 may also be formed as a bulbous shape that is uniform all about the closed end of the penis and not on just one side thereof.

Once penis 30 is properly adorned by condom 10, it may safely be inserted into a vagina during sexual intercourse. FIG. 6 illustrates a cross-sectional view of a female pelvic region 2 showing the relative locations of the pubic bone 3, clitoris 4, urethra 5, Grafenberg spot (G-spot) 6, and vagina 8. To commence intercourse, penis 30 will inwardly thrust into vagina 8 in the direction of arrow 7.

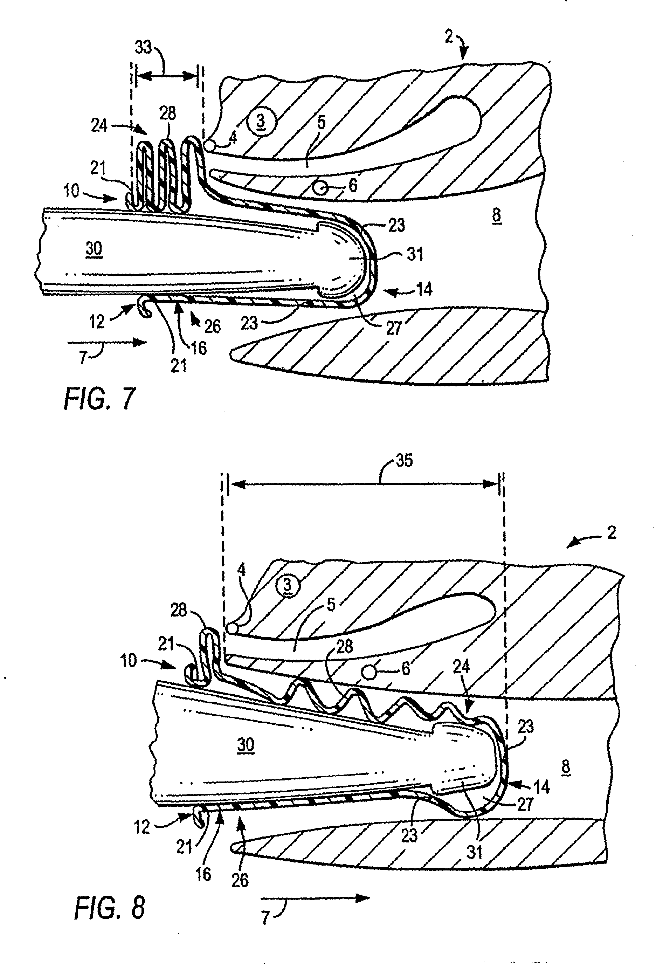

As shown in FIG. 7, as penis 30 is thrusted further into the canal of vagina 8 in the direction of arrow 7, certain folds 28 tend to contact the female anatomy external to and about vagina 8 and gather along the length of long side portion 24 between proximal end 21 of curved region 20 and the female anatomy. As the distance 33 between proximal end 21 of curved region 20 and the opening of vagina 8 gradually decreases due to the deeper penetration of penis 30 into vagina 8, the pressure created by folds 28 between proximal end 21 and the opening of vagina 8 gradually increases, such that at least some of the folds 28 must eventually pass from outside of vagina 8 into the vaginal canal. Preferably, folds 28 of condom 10 are oriented along the top of penis 30 with respect to female pelvic region 2, such that they are proximal to clitoris 4, as shown in FIGS. 6-9, for example. In this preferred orientation of condom 10 with respect to female pelvic region 2, the pressure created by folds 28 directly stimulates clitoris 4 during insertion of penis 30 into vagina 8.

As shown in FIG. 8, once the pressure has been released and some of the previously gathered folds 28 have passed from outside vagina 8 into the vaginal canal, they may once again spread out along the length of long side portion 24 due to any lubrication present, the pressure of the vaginal walls on the condom, and the elasticity of the condom itself, for example. This movement and dispersing of folds 28 along the length of curved region 20 provides stimulation not only to penis 30, but also to the interior walls of vagina 8, and preferably to G-spot 6.

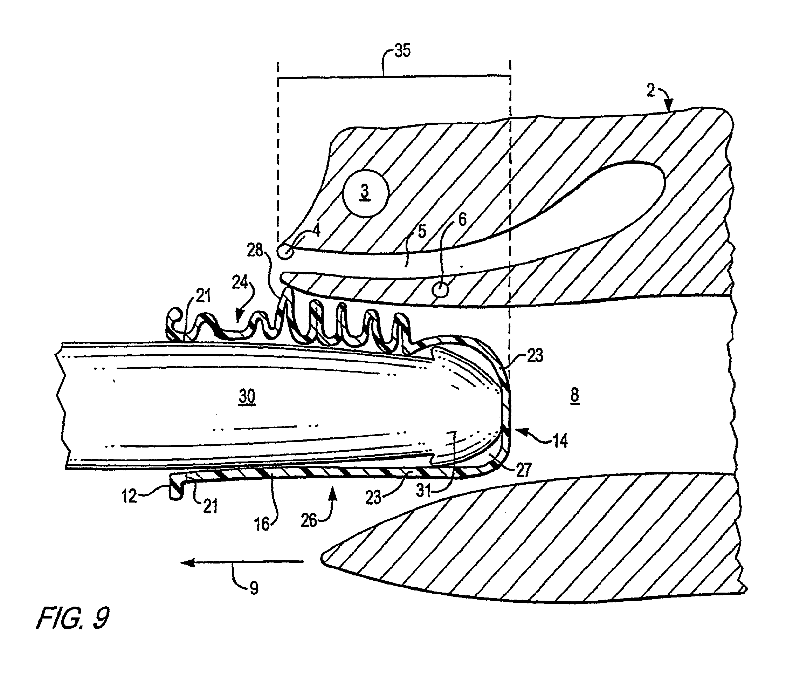

As shown in FIG. 9, as penis 30 is at least partially withdrawn from vagina 8 by an outward thrust in the direction of arrow 9, certain folds 28 tend to contact the female anatomy internal to and about vagina 8 and gather along the length of long side portion 24 between distal end 23 of curved region 20 and the female anatomy. As the distance 35 between distal end 23 of curved region 20 and the opening of vagina 8 gradually decreases due to the further withdrawal of penis 30 from vagina 8, the pressure created by folds 28 between distal end 23 and the opening of vagina 8 gradually increases, such that at least some of the folds 28 must eventually pass from inside the vaginal canal to outside of vagina 8. Again, preferably, folds 28 of condom 10 are oriented along the top of penis 30 with respect to female pelvic region 2, such that they are proximal to clitoris 4, and such that the pressure created by folds 28 not only stimulates the penis and the interior walls of vagina 8, but also directly stimulates clitoris 4 during not only each instroke of penis 30 into vagina 8 but also each outstroke of penis 30 from vagina 8.

Lubrication may preferably be provided on interior 17 of tubular body portion 16 to facilitate the in and out length-wise rubbing action of folds 28 and/or pocket 27 against the outer surface of penis 30 and to produce a hydrodynamic flushing thereof for enhancing the stimulation of the male user during sexual intercourse. Any suitable lubrication may be used for this purpose, but the type of lubrication preferably varies depending on the material used to form tubular body 16. For example, silicon oil lubricant may be preferable in conjunction with a tubular body made of latex, while mineralized oil may be the preferable lubrication for use with a thermoplastic tubular body. Lubrication may also preferably be provided on exterior 19 of tubular body 16 to provide for comfortable penetration of penis 30 into vagina 8 and to facilitate the in and out length-wise rubbing action of folds 28 against the interior walls of vagina 8 for enhancing the stimulation of the female user during sexual intercourse.

The increase in sensitivity attributable to the looseness, rubbing, and the previously described hydrodynamic action of pocket 27 and/or folds 28 all combine to enable the thickness of wall 18 of condom 10 to be increased to thicknesses on the order of 0.05-0.15 millimeters, while maintaining sensitivity equal to or greater than that found in ultra-thin condoms having wall thicknesses of about 0.03 millimeters. The ability to use greater wall thicknesses provides improved strength against rupture or tearing, which is important in the protection of a user against life-threatening diseases.

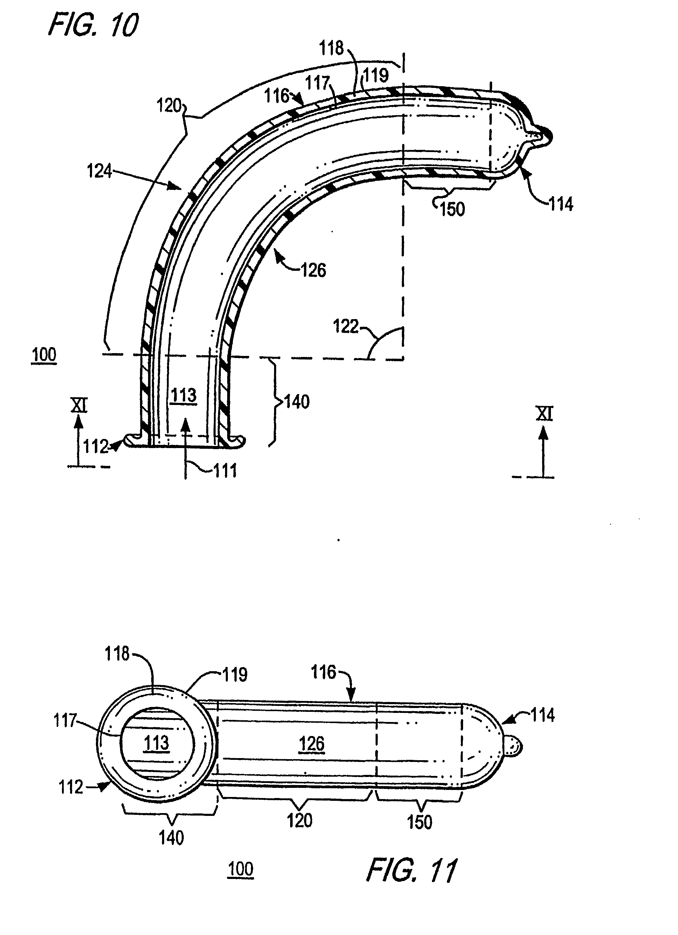

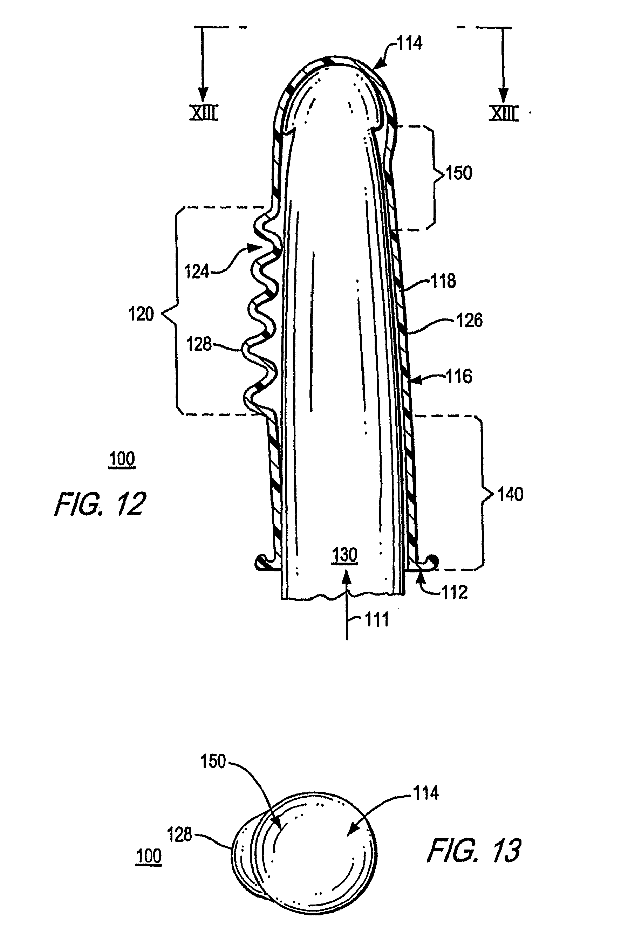

Although curved region 20 has been described above with respect to FIGS. 1-9 to extend longitudinally along the entire length of tubular body 16 between open end 12 and closed end 14, the curved region of the male condom may only extend along one or more portions of an otherwise straight tubular body, according to other embodiments of the invention. For example, as shown in FIGS. 10-13, condom 100 is provided with a curved region 120 along tubular body 116 between straight portions 140 and 150. Curved region 120 has a curvature angle 122, thereby defining a long side portion 124 and a short side portion 126 of wall 118, such that when a penis 130 is inserted into chamber 113 through open end 112 in the direction of arrow 111, one or more folds 128 are formed along long side portion 124 of wall 118, as shown in FIGS. 12 and 13.

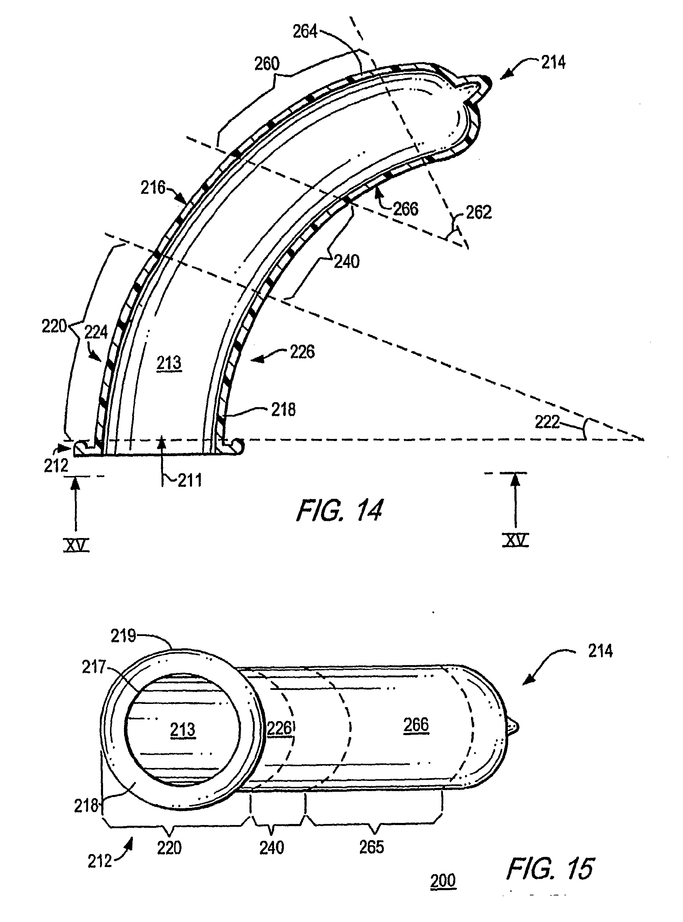

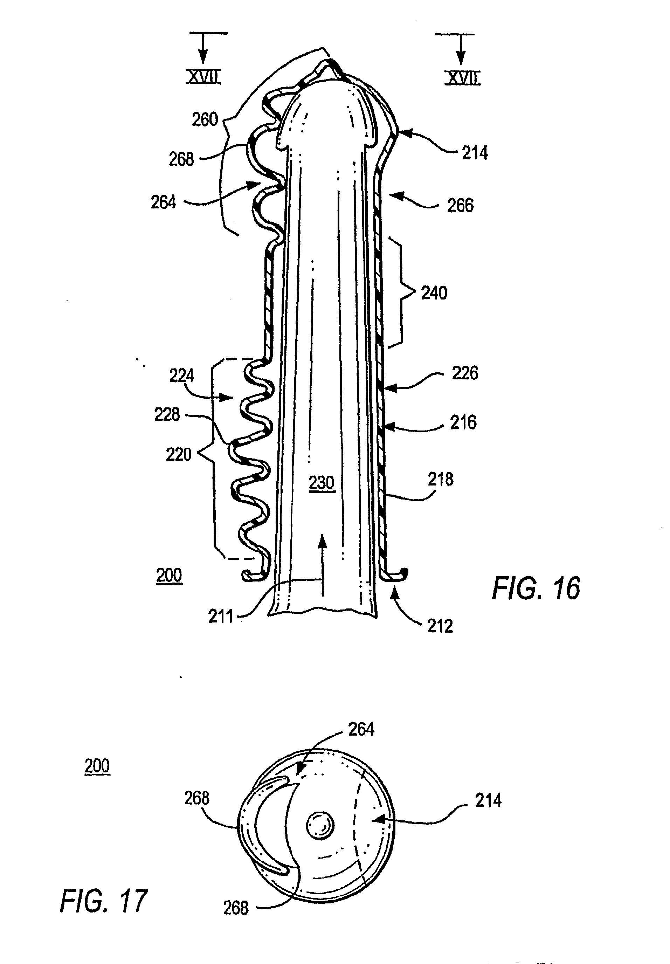

In yet another embodiment of the invention, instead of having only a single curved region along the tubular body (see, e.g., curved region 120 of condom 100), the condom may have multiple curved regions that create folds along different portions of the tubular body upon insertion of a penis therein. For example, as shown in FIGS. 14-17, condom 200 is provided with a first curved region 220 along tubular body 216 between open end 212 and straight portion 240, and a second curved region 260 along tubular body 216 between straight portion 240 and closed end 214. First curved region 220 has a curvature angle 222, thereby defining a first long side portion 224 and a first short side portion 226 of wall 218, and second curved region 260 has a curvature angle 262, thereby defining a second long side portion 264 and a second short side portion 266 of wall 218, such that when a penis 230 is inserted into chamber 213 through open end 212 in the direction of arrow 211, one or more folds 228 are formed along long side portion 224 of wall 218 and one or more folds 268 are formed along long side portion 264 of wall 218, as shown in FIGS. 16 and 17.

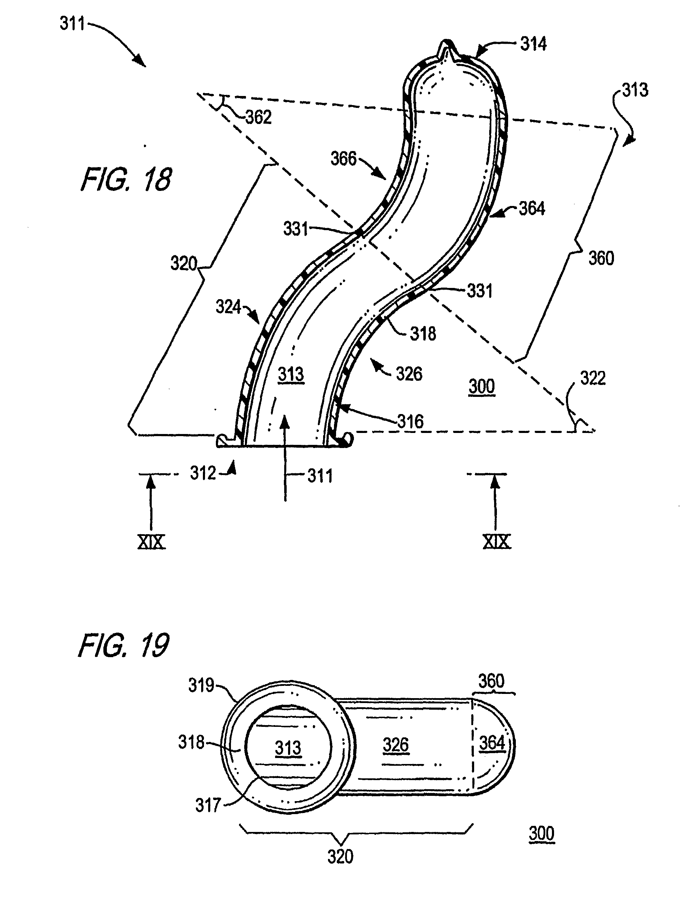

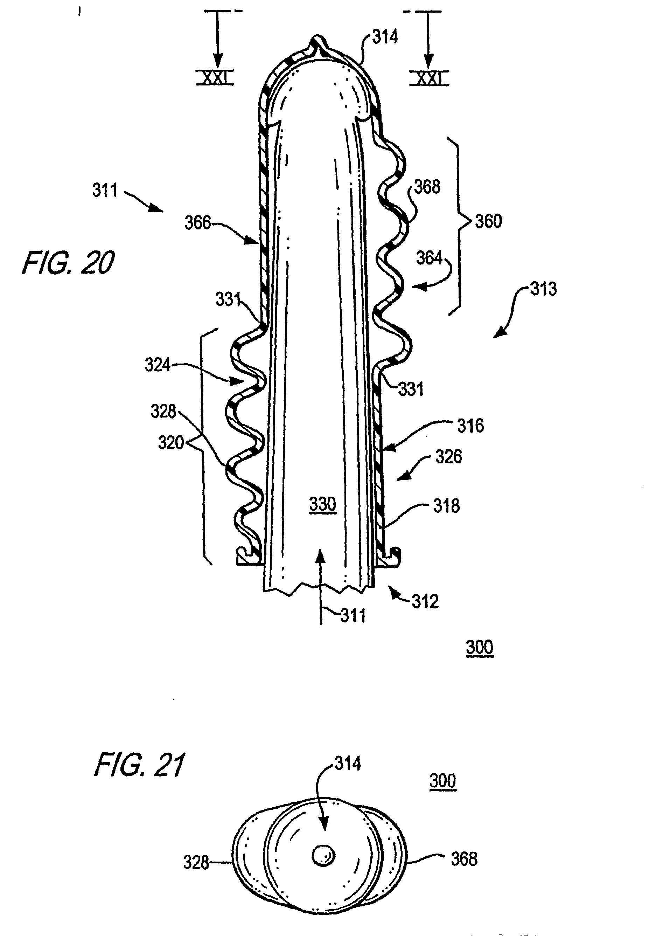

In yet another embodiment of the invention, instead of having multiple curved regions providing their respective long side portions, and therefore their respective folds, on substantially the same side of the tubular body (see, e.g., curved regions 220 and 260 of condom 200), the condom may have curved regions that create folds on opposite sides of the tubular body upon insertion of a penis therein. For example, as shown in FIGS. 18-21, condom 300 is provided with a first curved region 320 along tubular body 316 between open end 312 and a point 331, and a second curved region 360 along tubular body 316 between point 331 and closed end 314. First curved region 320 has a curvature angle 322, thereby defining a first long side portion 324 on a first side 311 of wall 318 and a first short side portion 326 on a second side 313 of wall 318, and second curved region 360 has a curvature angle 362, thereby defining a second long side portion 364 on second side 313 of wall 318 and a second short side portion 366 on first side 311 of wall 318, such that when a penis 330 is inserted into chamber 313 through open end 312 in the direction of arrow 311, one or more folds 328 are formed along long side portion 324 on first side 311 of wall 318 and one or more folds 368 are formed along long side portion 364 on second side 313 of wall 318, as shown in FIGS. 20 and 21.

In still yet another embodiment of the invention, instead of having multiple curved regions providing their respective long side portions, and therefore their respective folds, on substantially opposite sides of the tubular body in a zig-zag formation perpendicular to the condom's longitudinal axis (see, e.g., curved regions 320 and 360 of condom 300), the condom may have multiple curved regions placed all along and about the tubular body. For example, as shown in FIGS 22-27, condom 400 may be provided with multiple curved regions such that their respective long side portions, and therefore their respective folds, combine to create a continuous twist 495 about and along the entire condom from open end 412 to closed end 414, for example.

Condom 400 is provided with a first curved region 420 along tubular body 416 between open end 412 and a first point 431, a second curved region 460 along tubular body 416 between first point 431 and a second point 432, a third curved region 520 along tubular body 416 between second point 432 and a third point 433, and a fourth curved region 560 along tubular body 416 between third point 433 and closed end 414. First curved region 420 has a curvature angle 422, thereby defining a first long side portion 424 on a first side 411 of wall 418 and a first short side portion 426 on a second side 413 of wall 418. Second curved region 460 has a curvature angle 462, thereby defining a second long side portion 464 on a third side 415 of wall 418 and a second short side portion 466 on a fourth side 417 of wall 418. Third curved region 520 has a curvature angle 522, thereby defining a third long side portion 524 on second side 413 of wall 418 and a third short side portion 526 on first side 411 of wall 418. Fourth curved region 560 has a curvature angle 562, thereby defining a fourth long side portion 564 on fourth side 417 of wall 418 and a fourth short side portion 566 on third side 415 of wall 418, such that when a penis 430 is inserted into chamber 413 through open end 412 in the direction of arrow 411, one or more folds 428 are formed along long side portion 424 on first side 411 of wall 418, one or more folds 468 are formed along long side portion 464 on third side 415 of wall 418, one or more folds 528 are formed along long side portion 524 on second side 413 of wall 418, and one or more folds 568 are formed along long side portion 564 on fourth side 417 of wall 418, as shown in FIGS. 24-27, and such that all the folds combine to form continuous twist 495 about and along the entire condom from open end 412 to closed end 414.

The folds of each of the "zig-zag" embodiment of FIGS. 18-21 and the "twist" embodiment of FIGS. 22-27 allow the condoms to be elastic without requiring the wall of the tubular body to be made thinner than condoms currently available. Even when the condom is made with a tubular body whose wall thickness is over double that of currently available condoms, the "zig-zag" or "twist" shapes ensure that the condom stretches out and retracts during use. Besides acting on the full length of the condom, this stretchability and retractability may also be imparted between specific curve points along the length of the condom (e.g., points 431-433 of FIGS. 22-27).

The curvature angle of the curved region or regions may be of any suitable degree, but is preferably between 20 and 180 degrees. In the various embodiments of prophylactic devices of this invention, the sizes of the tubular bodies and the wall thicknesses of the materials may vary. The tubular body portion may preferably have a length on the order of 160-200 millimeters from its open end to its closed end, for example. Although the circumference of the tubular body is generally uniform, it could vary if desired. In the main body portion, the circumference is preferably about 95-105 millimeters, for example.

In certain embodiments of the invention, one or more stimulating devices may be provided within one or more pouches extending inwardly into, along, or perpendicular to the chamber defined by the tubular body of the condom for providing stimulation to one or more sexual organs of one or both of the partners, thereby enhancing the pleasure felt during sexual intercourse. Various types of stimulating devices may be utilized at various locations on the male condom for stimulating specific sexual organs in specific ways.

In a particularly preferred embodiment of the invention, a stimulating device may be fastened within a pouch that extends inwardly into the chamber of the condom of the invention such that the stimulator aligns with the G-spot during sexual intercourse. As illustrated in FIGS. 28-30, similarly to condom 10, for example, a male condom 1010 of the invention has an open end 1012, a closed end 1014, and a tubular body portion 1016 that extends longitudinally between open end 1012 and closed end 1014 to define a chamber 1013. Tubular body 1016 has a thin membrane wall 1018 with an interior surface 1017 and an exterior surface 1019. Male condom 1010 may be formed with one or more curved regions, as described above with respect to condoms 10, 100, 200, 300, and 400, however condom 1010 is illustrated as being substantially straight for the sake of clarity of the drawings. In fact, stimulating devices can be integrated into any of the various types of condoms of the invention, including straight-walled, bulbed, spring-action, or curved, for example.

With continued reference to FIG. 28, one or more pouches 1070 having an open end 1072 formed in wall 1018 of tubular body 1016, a closed end 1074, and a tubular pouch body 1076 that extends longitudinally between open end 1072 and closed end 1074 to define a pouch chamber 1073 for receiving a stimulating device therein may be provided. Tubular pouch body 1076 preferably extends into chamber 1013 of tubular body 1016 and has a thin membrane wall 1078 with an interior surface 1077 and an exterior surface 1079. Tubular pouch body 1076 may be formed from any suitable elastic and impermeable membrane material, such as natural rubber (e.g., latex), synthetic rubber (e.g., silicone rubber or neoprene), polyurethane, and other elastic substances (e.g., thermoplastic polyolefin elastomers), for example, and is preferably formed from the same material as tubular body 1016.

As illustrated in FIG. 29, tubular pouch body 1076 extends into chamber 1013 of tubular body 1016, such that when a penis (e.g., penis 1030) is inserted into chamber 1013 of condom 1010 in the direction of arrow 1011, tubular pouch body 1076 is held in place between the external surface of the penis and interior 1017 of wall 1018. In certain embodiments of the invention, preferably after a user has inserted his penis into chamber 1013, he may insert a stimulating device (e.g., stimulating device 1080) into chamber 1073 of tubular pouch body 1076. Therefore, stimulator 1080 may be positioned within chamber 1073 so as to provide continuous stimulation to penis 1030 for the entire period of time condom 1010 is in use.

As shown in FIGS. 29 and 30, stimulator 1080 may include a stimulating head portion 1082 external to condom 1010 and an extension flap 1084 extending from head portion 1082 into and along chamber 1073 for promoting the stimulation provided by head portion 1082 to various other parts of the condom. Head portion 1082 may provide contact with and stimulate clitoris 4 during sexual intercourse, while extension flap 1084 may provide stimulation to the internal walls of vagina 8, and preferably G-spot 6. Furthermore, as described above, tubular pouch body 1076 may be held in place between the external surface of penis 1030 and interior 1017 of wall 1018, such that when extension flap 1084 is positioned within chamber 1073, penis 1030 may seem to have more girth, thereby providing more pleasure to the female genitalia during sexual intercourse.

Stimulator 1070 may be retained within chamber 1073, as described above, by various means. In one embodiment, as shown in FIGS. 29 and 30, for example, extension flap may have a narrow portion 1083 that may form a secure fit with opening 1072 of chamber 1073, thereby locking stimulator 1080 in place with respect to condom 1010, for example.

Although only one pouch 1070 has been shown in the illustrative embodiment of FIGS. 28-30, condom 1010 may be provided with multiple pouches 1070 having openings 1072 at various locations about wall 1018 of tubular body 1016, and extending to various lengths within chamber 1013, for retaining various types of stimulating devices in order to provide stimulation to one or more sexual organs of one or both of the partners, thereby enhancing the pleasure felt during sexual intercourse.

The male condom of the invention may be provided with one or more stimulation devices that can provide stimulation to one or more sexual organs of one or both of the partners for enhancing the pleasure felt during sexual intercourse. Stimulators of the invention, such as stimulator 1080, may either be an electronic device (e.g., a battery operated vibrator) or simply a mechanical device (e.g., a flexible rubber element with protrusions) that is able to provide stimulation to a sexual organ during sexual intercourse.

In a preferred embodiment of the invention, any electronic device provided with the male condom as a stimulator preferably works on the principle of making physical contact with the user through a moving object that offers impact with the least resistance. This type of stimulator, unlike common vibrators, does not have an eccentric weight attached to a motor shaft, but instead preferably has an offset shaft attached to a rotor. The shaft can be a singular or multiple offset or non-offset shaft, and attached to the one or more shafts is preferably one or more sleeves that are able to move freely when mounted on its shaft. A power source, the motor, and its shaft or shafts are preferably contained within an outer case, and one or more openings are preferably provided in the case to enable direct contact between the sleeves and a sexual organ for the stimulation thereof.

When the motor is in operation in this preferred embodiment, each sleeve may spin around the rotor shaft and, due to centrifugal force, may be outwardly displaced until it makes contact with a fixed object (e.g., the sexual organ to be stimulated). Upon hitting the fixed object, the sleeve may preferably be forced to lessen its contact with the object, either by the sleeve deforming, by the sleeve sliding about its shaft, or by the shaft sliding itself, until the sleeve is freed of contact with the object. Once freed of contact with the fixed object, the sleeve may preferably return to its outward displacement while spinning about its shaft. This movement of the sleeve from being displaced outwardly, to being freed from contact with the fixed object, to returning to its outward displacement allows for the stimulator to contact a fixed object during every rotation of the shaft while offering the least resistance. As this contact is made with the least resistance, the energy required by the stimulator is very low and the contact with the fixed object is non-vibratory in nature.

In one embodiment, as shown in FIGS. 31A and 31B, a stimulator 1100 of the invention has an outer case 1101 containing therein a motor 1104 that is coupled to a rotor assembly 1103 and a power source 1105. Rotor assembly 1103 preferably includes a double offset shaft fixture 1106 mating the rotor of motor 1104. Each offset shaft 1106 preferably has one or more outwardly displaceable sleeves 1107 that may be held in position thereon by one or more stoppers 1108. When motor 1104 starts drawing power from power source 1105, the rotor may start rotating shafts 1106 to let sleeves 1107 occupy their outward position, thereby preferably forming a fan-like structure (see, e.g., FIG. 31B), which has restricted access through openings 1102 in case 1101 to provide direct contact with a sexual organ for stimulation thereof.

An alternative embodiment of a stimulator 1110 is shown in FIG. 32, where a motor 1111 is connected to a power source 1112 and a rotor assembly 1113 by a non-offset shaft 1114 extending out to accommodate a sleeve 1115. Sleeve 1115 may be hollow and may move outwardly through opening 1116 in case 1117 as the rotor starts rotating, but may be prevented from moving completely off of shaft 1114 by a stopper 1118. Additionally, the end of shaft 1114 may extend from a slot 1119, whereby as the shaft rotates, sleeve 1115 rotates but will cause shaft 1114 to slide back along slot 1119 upon impact with the anatomy to be stimulated.

In certain preferred embodiments, the sleeves are provided as flexible pads that are integral parts of a shaft, such that as the shaft rotates, the flexible pads rotate but will bend or give way to resistance upon impact with the anatomy to be stimulated. Any suitable material may be used to form these flexible pads or any of the sleeves described above, such as foam, PVC, rubber, polypropylene, polyurethane, or any combination thereof, for example. Extensions, such as bristles 1120 shown in FIG. 32, may be formed upon each sleeve to provide the flexibility for impacting the anatomy to be stimulated with the least resistance.

In accordance with other aspects of the invention, a male condom of the invention is formed by a process as set forth in FIGS. 33-35.

The process includes a mold 1220 having a handle 1222 and a protuberance 1224 extending outwardly therefrom. Protuberance 1224 has an outer surface 1226 thereon dimensioned to correspond to that of the inside surface of the condom and any additional pouches to be made.

In practicing the invention, the mold 1220 is connected to suitable carriage means for movement into an open ended container 1228 that has a body of synthetic or latex material 1230 therein of a composition as discussed in the earlier embodiments, which can be deposited on surface 1226 to form a resultant thin layer deposit thereon. The deposit can be formed by a single or double dip process which includes directing mold 1220 along a path into the body of material 1230 to form a layer of material on surface 1226.

All conventional continuous dipping and molding of substantially straight condoms is done by dipping the mold 1220 into the body of material 1230 such that the tip of the closed end of the condom enters into the body of material, parting the coating material, and thereby causing minimum disturbance and preventing any air bubbles from getting trapped therein. This is followed by the body extending to the required length of the condom to be manufactured. In this dipping technique, only one coating is possible per dip tank and therefore any imperfections in the coating has to be corrected in a second dipping tank using additional coating layers.

Controlled angular dipping should preferably be carried out for asymmetrically shaped prophylactic devices (e.g., spiral condom 400). This type of condom needs to be dipped at an angular entry (e.g., between 30 and 45°degrees) and at a controlled slow rotation to ensure that the coating material moves around the longitudinal axis of the mold to obtain an even spread of the coating material. Here again, the coating material ensures only one coating. However imperfection in the coating can be partially correct by the material flow around the mould until the time the material gels.

In dipping both symmetrical and asymmetrical (e.g., spiral) shapes to obtain an even more uniform coating of material with negligible pin holes, it is best to dip such that one half of the rotating mold is within the coating material at all times. This will ensure that with a controlled rotation, the coating material continuously coats a layer and imperfections, if any, in the coating will get corrected with each subsequent rotation and coating. All this can be achieved without adding a thick coating layer.

The resultant deposited material is shown in FIG. 34. The deposited material is stripped from the mold 1220 by relative movement between the open end 1232 and the flat surface of mold handle 1222, whereby the open end 1232 assumes the dotted line position. This causes the pouch portion of the prophylactic device to be telescoped upon itself to form a double wall telescoped segment 1238. The double walled telescope segment 1238 is then engaged by suitable means such as rollers 1240 to roll the telescoped double wall portion 1238 upon itself from a return bend portion 1242. As shown in FIG. 35, this rolled ring 1248 exposes the end closure 1246 of the prophylactic device. The rolling of the ring 1248 will remove the prophylactic device from the form 1220. The removed prophylactic device can then be readily manipulated for various forms of packaging and storage.

With continued reference to FIGS. 28-30, open end 1072 of pouch 1070 may be provided near open end 1012 of the condom and pouch 1070 may be provided with a stimulating device therein upon manufacture of the condom, such that when rolled for packaging, the stimulating device may already be secured therein. The user may insert his penis through the open end and roll the condom down along his penis a preferred length, preferably such that open end 1072 of pouch 1070 is still within the rolled ring of the condom to prevent the open end from being exposed and from allowing the stimulating device to fall out of its chamber 1073, for example.

In yet another particularly preferred embodiment of the invention, a stimulating device may be fastened within a pouch that extends externally along the chamber of the condom of the invention such that the stimulator aligns with the G-spot during sexual intercourse. As illustrated in FIGS. 36-38, similarly to condom 10, for example, a male condom 1310 of the invention has an open end 1312, a closed end 1314, and a tubular body portion 1316 that extends longitudinally between open end 1312 and closed end 1314 to define a chamber 1313. Tubular body 1316 has a thin membrane wall 1318 with an interior surface 1317 and an exterior surface 1319. Male condom 1310 may be formed with one or more curved regions, as described above with respect to condoms 10, 100, 200, 300, and 400, however condom 1310 is illustrated as being substantially straight for the sake of clarity of the drawings.

With continued reference to FIG. 36, one or more pouches 1370 having an open end 1372, a closed end 1374, and a tubular pouch body 1376 that extends longitudinally between open end 1372 and closed end 1374 to define a pouch chamber 1373 for receiving a stimulating device therein may be provided. Tubular pouch body 1376 preferably extends external to and along tubular body 1316 and has a thin membrane wall 1378 with an interior surface 1377 and an exterior surface 1379. Wall 1378 may share common material with wall 1318 along their common areas. Tubular pouch body 1376 may be formed from any suitable elastic and impermeable membrane material, such as natural rubber (e.g., latex), synthetic rubber (e.g., silicone rubber or neoprene), polyurethane, and other elastic substances (e.g., thermoplastic polyolefin elastomers), for example, and is preferably formed from the same material as tubular body 1316.

As illustrated in FIG. 37, tubular pouch body 1376 extends parallel to chamber 1313 of tubular body 1316, such that when a penis (e.g., penis 1330) is inserted into chamber 1313 of condom 1310 in the direction of arrow 1311, tubular pouch body 1376 is external to chamber 1313, and open end 1372 is preferably retained within the rolled ring such that stimulating device 1380 may be provided within condom 1310 on manufacture and won't escape chamber 1373 during sexual intercourse. Therefore, stimulator 1380 may be positioned within chamber 1373 so as to provide continuous stimulation to penis 1330 for the entire period of time condom 1310 is in use.

As shown in FIGS. 36-38, chamber 1373, and thus stimulator 1380, may extend along the length of the condom for a length 1393 and about some or all of the circumference of the condom by length 1395, depending upon the one or more sexual organs desired to be stimulated by the stimulator.

Thus, it is seen that a male condom having at least one curved region has been provided that is capable of forming one or more folds for stimulating a sexual organ during sexual intercourse. One skilled in the art will appreciate that the invention can be practiced by other than the described embodiments, which are presented for purposes of illustration and not of limitation, and the invention is limited only by the claims which follow.

The disclosure of this application also includes the following number of clauses:
1. A male condom, said condom comprising:
   an open end, a closed end, and a tubular body extending longitudinally between said open end and said closed end to define a chamber, wherein said tubular body has a wall with an interior surface and an exterior surface, wherein said tubular body has at least one curved region to define a long side portion and a short side portion of said wall, and wherein said tubular body is capable of forming one or more folds along said long side portion of said wall for stimulating a sexual organ during sexual intercourse on insertion of a penis into said chamber.
2. The condom of clause 1, wherein said curved region extends from said open end to said closed end.
3. The condom of clause 1, wherein said tubular body has multiple curved regions to provide extended elasticity during sexual intercourse.
4. The condom of clause 1, wherein said exterior of said tubular body is lubricated to facilitate sliding of said one or more folds.
5. The condom of clause 1, wherein said interior of said tubular body is lubricated to facilitate sliding of said one or more folds.
6. A male condom, said condom comprising:
   a first pouch having a first open end, a first closed end, and a first tubular body extending longitudinally between said first open end and said first closed end to define a first chamber, wherein said first tubular body has a first wall with a first interior surface and a first exterior surface; and
   a second pouch having a second open end formed in said first wall of said first tubular body, a second closed end, and a second tubular body extending longitudinally between said second open end and said second closed end to define a second chamber for receiving a stimulating device, wherein said second tubular body has a second wall with a second interior surface and a second exterior surface, wherein said second pouch extends inwardly into said first chamber, and wherein said stimulating device is capable of stimulating a penis on insertion of said penis into said first chamber.
7. A male condom, said condom comprising:
   a first pouch having a first open end, a first closed end, and a first tubular body extending longitudinally between said first open end and said first closed end to define a first chamber, wherein said first tubular body has a first wall with a first interior surface and a first exterior surface; and
   a second pouch having a second open end formed, a second closed end, and a second tubular body extending longitudinally between said second open end and said second closed end to define a second chamber for receiving a stimulating device, wherein said second tubular body has a second wall with a second interior surface and a second exterior surface, wherein said second pouch extends parallel to said first chamber along said first wall, and wherein said stimulating device is capable of stimulating a penis on insertion of said penis into said first chamber.
8. The condom of clause 7, wherein said stimulating device and said condom is rolled such that a user is capable of unrolling said condom over said penis to a length for sexual intercourse, wherein said second open end is rolled when said condom is unrolled to said length.
9. The condom of clause 7, wherein said second chamber is non-circular about said first tubular body.

## Claims

1. A male condom, said condom comprising:
a first pouch having a first open end, a first closed end, and a first tubular body extending longitudinally between said first open end and said first closed end to define a first chamber, wherein said first tubular body has a first wall with a first interior surface and a first exterior surface; and
a second pouch having a second open end formed in said first wall of said first tubular body, a second closed end, and a second tubular body extending longitudinally between said second open end and said second closed end to define a second chamber for receiving a stimulating device, wherein said second tubular body has a second wall with a second interior surface and a second exterior surface, wherein said second pouch extends inwardly into said first chamber, and wherein said stimulating device is capable of stimulating a penis on insertion of said penis into said first chamber.

2. A male condom, said condom comprising:
a first pouch having a first open end, a first closed end, and a first tubular body extending longitudinally between said first open end and said first closed end to define a first chamber, wherein said first tubular body has a first wall with a first interior surface and a first exterior surface; and
a second pouch having a second open end formed, a second closed end, and a second tubular body extending longitudinally between said second open end and said second closed end to define a second chamber for receiving a stimulating device, wherein said second tubular body has a second wall with a second interior surface and a second exterior surface, wherein said second pouch extends parallel to said first chamber along said first wall, and wherein said stimulating device is capable of stimulating a penis on insertion of said penis into said first chamber.

3. The condom of claim 2, wherein said stimulating device and said condom is rolled such that a user is capable of unrolling said condom over said penis to a length for sexual intercourse, wherein said second open end is rolled when said condom is unrolled to said length.

4. The condom of claim 2, wherein said second chamber is non-circular about said first tubular body.
